# EUROPÄISCHE PATENTSCHRIFT

(11) **EP 1 370 159 B1**
(45) Veröffentlichungstag und Bekanntmachung des Hinweises auf die Patenterteilung: **23.01.2008**
(21) Anmeldenummer: 02706507.7
(22) Anmeldetag: 19.03.2002
(51) Int. Cl.: A23L 1/30, A61K 36/899, A23L 2/02, A61K 36/00

(54) **VERFAHREN ZUR HERSTELLUNG VITAMIN B-ANGEREICHERTER PRÄPARATE AUS GRÄSERN**
METHOD FOR PRODUCING VITAMIN B-ENRICHED PREPARATIONS FROM GRASSES
PROCEDE DE PRODUCTION DE PREPARATIONS ENRICHIES EN VITAMINE B A PARTIR DE GRAMINEES

(30) Priorität: 21.03.2001 AT 4502001
(43) Veröffentlichungstag der Anmeldung: 17.12.2003
(73) Patentinhaber: vis-vitalis Lizenz- und Handels GmbH, 5081 Anif (AT)
(72) Erfinder: SADEGHI, Behzad, A-1100 Wien (AT); KÖSSLER, Peter, A-5571 Mariapfarr (AT); FUCHS, Norbert, A-5571 Mariapfarr (AT)
(74) Vertreter: Sonn & Partner Patentanwälte
(86) Internationale Anmeldenummer: PCT/AT2002/000089
(87) Internationale Veröffentlichungsnummer: WO 2002/074103

(56) Entgegenhaltungen:
- US-A- 5 554 445
- US-A- 5 820 916
- MOZAFAR A. ET AL: "Uptake and Transport of Thiamin (Vitamin B1) by Barley and Soybean" J. PLANT PHYSIOL., Bd. 139, 1992, Seiten 436-442, XP001051110
- DATABASE WPI Section PQ, Week 199445 Derwent Publications Ltd., London, GB; Class P11, AN 1994-365041 XP002204585 & SU 1 825 294 A (UKR IRRIGATED FARMING RES INST), 30. Juni 1993 (1993-06-30) in der Anmeldung erwähnt
- DATABASE WPI Section PQ, Week 199312 Derwent Publications Ltd., London, GB; Class P11, AN 1993-099262 XP002204586 & SU 1 727 600 A (SARAT AGRIC MECHN INST), 23. April 1992 (1992-04-23) in der Anmeldung erwähnt

## Beschreibung

Die Erfindung betrifft ein Verfahren zur Herstellung Vitamin B-angereicherter Präparate aus Gräsern.

Vitamine des B-Komplexes finden vielfältige Anwendungen im Bereich der pharmazeutischen Industrie ebenso wie im Bereich der Lebensmittelindustrie. Während allerdings für bestimmte Vitamine (z.B. Vitamin C, Vitamin E) eine Reihe natürlicher Rohstoffe zur Herstellung Vitamin C-reicher Präparate (z.B. Acerola-Kirschen, Hagebuttenfrüchte, Ölauszüge aus Weizenkeimen und anderen Getreiden für natürliche Vitamin E-Rohstoffe) zur Verfügung stehen, werden Vitamine des B-Komplexes vorwiegend synthetisch und/oder auf mikrobiologischem Wege hergestellt. Derartige synthetische oder mikrobiologische Herstellungsweisen sind jedoch nicht unproblematisch, vor allem hinsichtlich von Kontaminationen mit störenden Substanzen, die im Zuge des Syntheseweges in das Präparat gelangen. Auch liegen die Vitamine des B-Komplexes, beispielsweise bei der Herstellung durch (manipulierte) Mikroorganismen in Kombination mit Substanzen vor, mit denen sie natürlicherweise nicht, z.B. in Pflanzen, vorkommen.

Weiters werden auch für Tier, Mensch und Pflanze essentielle Spurenelemente wie z.B. Selen oder Molybdän im Rahmen pharmazeutischer oder lebensmitteltechnologischer Zubereitungen häufig in anorganischer oder organisch-synthetischer Form eingesetzt.

Erwünscht ist aber sowohl für Vitamin B-Präparate als auch für Präparate, enthaltend essentielle Spurenelemente, dass derartige Präparate in effizienter Form vorliegen, vorzugsweise in Kombination mit den natürlichen Stoffwechselpartnern, mit denen sie in pflanzlichen Organismen vorkommen.

Die SU 1 825 294 A3 betrifft Süßklee- oder Sudangras-Mischungen mit Mais, wobei zwei Mähphasen angegeben werden, nämlich eine bei 175 bis 190 cm und eine bei 135 bis 140 cm. Bei diesem Verfahren geht es darum, proteinreiches Futtergras durch spezielle Saat-und Mähbedingungen zu gewinnen. Auf den Gehalt an Mikronährstoffen, wie z.B. B-Vitaminen oder Spurenelementen, wird in diesem Dokument nicht eingegangen.

Gemäß der SU 1 727 600 A1 wird ein vorzeitiger Mähschritt ("preliminary mowing") von Gräsern vor der Knospenformierung bei einer Höhe von etwa 12 bis 18 cm vorgesehen. Bei diesem Verfahren geht es nur darum, durch zweimaliges Mähen den Gras- bzw. Fruchtertrag pro definierter Anbaufläche zu steigern. Eine Beeinflussung des Vitamingehaltes durch mehrmaliges Mähen wird in diesem Verfahren weder beschrieben noch nahegelegt.

Gemäß der RU 2 108 731 C wird ein Grasfuttermaterial hergestellt, wobei Koagulierungsmittel und Konservierungsmittel zugegeben werden. Hierbei werden lediglich mechanische und chemische Methoden beschrieben, mit denen nährstoffreiche Tierfuttermittel produziert werden sollen.

Die US 5 773 681 A betrifft die Herstellung von Elektrolyt-angereicherten Keimlingen. In diesem Dokument wird eine Methode beschrieben, um keimfähige Samen ohne Einbringung in den Boden mit Elektrolyten anzureichern. Dieses Dokument enthält keinerlei Angaben zur Mäh-Strategie von Gräsern bzw. zum Vitamin B-Gehalt von Gräsern in Abhängigkeit von bestimmten Schnittstrategien.

Die EP 0 652 707 B1 betrifft die Herstellung von inkrustierten Samen, wobei es vor allem darum geht, Nährstoffe wie Stickstoff, Phosphor, Kalium und/oder Mikroelemente unter Verwendung von Chitosan (einem natürlichen Polymerisat aus Meereskrustentieren) auf Saatgut aufzubringen.

US-A-5 820 916 offenbart ein Verfahren zur Herstellung eines Nahrungs-Supplementsaus Weizengrass, welches die Ernte kurz vor der Blattbildung umfaßt.

MOZAFAR A. ET AL: "Uptake and Transport of Thiamin (Vitamin B1) by Barley and Soybean" J. PLANT PHYSIOL., Bd. 139, 1992, Seiten 436-442 offenbart Untersuchungen zur Aufnahme von Thiamin durch die Wurzeln und die Weiterverteilung in die oberirdischen Pflanzenteile.

In all diesen bekannten Verfahren wird jedoch weder der Vitamin B-Gehalt der Pflanzen untersucht, noch eine Erhöhung des Vitamin B-Gehaltes zu erzielen versucht.

Aufgabe der vorliegenden Erfindung ist daher ein Verfahren zu entwickeln und Techniken anzuwenden, um hochkonzentrierte Vitamin B-Komplexe aus natürlichen Rohstoffen zu gewinnen. Diese Verfahren sollten in standardisierbarer und reproduzierbarer Form industriell einsetzbar sein. Weiters sollten diese Vitamin B-Komplex-Präparate aus natürlichen Rohstoffen, bevorzugterweise im Hinblick auf ihren Gehalt an anorganischen Spurenelemente-Verbindungen, verbessert und im Endpräparat organisch verfügbar sein.

Gegenstand der vorliegenden Erfindung, mit welcher ein oder mehrere dieser Aufgaben gelöst werden kann, ist demgemäß ein Verfahren zur Herstellung Vitamin B-angereicherter Präparate aus Gräsern, bei welchem Gräser bis maximal vor der Knospungsphase wachsen gelassen werden, die Gräser geschnitten werden, die Gräser erneut wachsen gelassen werden, die Gräser erneut geschnitten werden, wobei der erneute Schnitt gesammelt wird, gegebenenfalls der Schritt des erneuten Wachsenlassens und erneuten Schneidens zumindest einmal wiederholt wird und die weiteren erneuten Schnitte gesammelt und vereinigt werden, und der erneute Schnitt oder die erneuten Schnitte zu einem Vitamin B-angereicherten Gräser-Präparat aufgearbeitet werden.

Wesentlich ist, dass vor dem ersten Schnitt das Wachstum nur bis zu einer Wachstumshöhe, bevor der Zustand der Knospung erreicht ist, erfolgt und spätestens zu diesem Zeitpunkt der erste Schnitt erfolgt. Dies ist insoferne wichtig, als nach Erreichen des Knospungs-zustandes ein 2. oder gar 3. Austreiben der Pflanzen nicht mehr möglich ist. In der Regel liegt die erwähnte Wachstumshöhe (kurz vor der Knospung) bei Weizengräsern bei etwa 20 - 30 cm. Andere Gräser bzw. erfindungsgemäß zu verwendende Pflanzen können entsprechend niedrigere (Luzerne) oder höhere Wachstumshöhen erreichen, bevor sie das Knospungs-Stadium erreichen.

Es stellte sich erfindungsgemäß überraschenderweise heraus, dass der Vitamingehalt, insbesondere der Gehalt an Vitamin B1 und Vitamin B2, in den erneuten Schnitten von Gräsern signifikant gegenüber dem Erstschnitt angereichert war und somit Vitamin B-Präparate erhalten werden können, die einen überaus hohen Gehalt an B-Vitaminen aufweisen. Darüberhinaus liegen die B-Vitamine in den erfindungsgemäß hergestellten Präparaten in Kombination mit vielen natürlichen pflanzlichen Interaktionspartnern vor; es fehlen natürlich auch jegliche Verunreinigungen, die durch chemische Synthese oder durch mikrobiologische Prozesse ins Präparat gelangen konnten (Katalysatoren-Rückstände, rekombinantes, mikrobielles oder sonstiges genetisch fremdes Material, ....).

Bevorzugterweise wird das erfindungsgemäße Verfahren mit Gräsern durchgeführt, die aus Elektrolyt-inkrustiertem Saatgut wachsen gelassen werden. Bei der Inkrustierung wird das Saatgut mit einer Düngelösung behandelt, die essentielle Spurenelemente, wie z.B. Selen, Molybdän, Chrom, Mangan, Kupfer, Ammonium oder Zink enthält. Bevorzugterweise wird dabei die Lösung auf die Oberfläche des Saatgutes aufgesprüht und mittels Bindemitteln.an der Oberfläche fixiert.

Weiters können bevorzugt auch elektrolytisch angereicherte Keimlinge, wie sie beispielsweise in der EP 0 770 324 A2 beschrieben sind, als Ausgangsmaterial für die Gräser verwendet werden.

Die so herangezogenen Gräser weisen einen gegenüber den mit üblichem Leitungs- oder Regenwasser inkubierten Saatgut bzw. Keimlingen verbesserten Gehalt an Spurenelementen, wie Chrom, Mangan, Eisen, Kupfer, Selen, Molybdän, etc., auf.

Bevorzugterweise wird das Elektrolyt-inkrustierte Saatgut durch Behandlung von Saatgut mit einer Inkrustierlösung enthaltend Natriumselenat, Natriummolybdat, Chrom-III-chlorid, Manganchlorid, Kupfergluconat, Ammonium-Eisen-III-citrat, Zinkgluconat oder Mischungen davon, erhalten, wobei die Konzentration dieser Salze bevorzugterweise im Bereich zwischen 0,1 mg bis 10 g/200 g Inkrustierlösung gewählt wird. Bevorzugterweise beträgt die Konzentration, jeweils pro 200 g Inkrustierlösung, von Natriumselenat 0, 1 bis 100 mg, insbesondere 1 bis 50 mg, von Natriummolybdat 0,1 bis 100 mg, insbesondere 1 bis 50 mg, von Chrom-III-chlorid 0,5 bis 500 mg, insbesondere 1 bis 100 mg, von Manganchlorid 1 bis 1000 mg, insbesondere 10 bis 500 mg, von Kupfergluconat 1 bis 2000 mg, insbesondere 10 bis 1000 mg, von Ammonium-Eisen-III-citrat 1 bis 2000 mg, insbesondere 10 bis 1000 mg, von Kupfergluconat 1 bis 2000 mg, insbesondere 10 bis 1000 mg, und von Zinkgluconat 1 bis 2000 mg, insbesondere 10 bis 1000 mg.

Die vorliegende Erfindung ist zur Erhöhung des Vitamin B-Gehaltes in allen Vitamin B-hältigen Gräsern geeignet. Bevorzugterweise werden erfindungsgemäß Gräser von Hülsenfrüchten und Getreidesamen behandelt. Dabei besonders bevorzugt sind Weizen-, Buchweizen-, Quinoa-, Mungobohnen-, Bockshornklee-, Rettich-, Alfalfa-, Mais-, Kürbis-, Roggen-, Gerste-, Reis-, Adzuki-Bohnen-, Erbsen-, Hirse-, Kichererbsen-, Kresse-, Leinsamen-, Linsen-, Senf-, Sesam-, Sojabohnen-, Sonnenblumen- und Amaranthgräser, insbesondere Weizengräser.

Bevorzugterweise wird das erhaltene Vitamin B-angereicherte Gräserpräparat zu Grassaft oder Grassaftpulver aufgearbeitet, welches dann vorwiegend im pharmazeutischen oder im Lebensmittelbereich weiter eingesetzt werden kann.

Vorzugsweise wird der erste Schnitt der Gräser bei einer Höhe von etwa 20 bis 30 cm durchgeführt (da bei dieser Wachstumshöhe bei den meisten kommerziell zu nützenden (Weizen-)Gräsern die Knospung noch nicht eingetreten ist), wobei bevorzugterweise auf einen Bodenabstand von unter 15, insbesondere unter 10 cm, geschnitten wird (im Mittel). Die erneuten Wachstumsschritte können dann ebenfalls bevorzugt auf eine Höhe von über 20 cm erfolgen, wobei die erneuten Schnitte analog zum ersten Schnitt durchgeführt werden können. Die geschnittenen Gräser können dann zu Saft gepresst und/oder sprühgetrocknet werden und dann vor allem in Form eines Grassaftpulvers über längere Zeit gelagert werden. Auch der Pressrückstand (der Presskuchen) stellt ein Vitamin B-angereichertes Gräserpräparat im Rahmen der vorliegenden Erfindung dar, welches einen erhöhten Vitamin B-Gehalt aufweist.

Gegenstand der vorliegenden Erfindung ist auch ein Vitamin B-angereichertes Gräserpräparat, welches nach dem erfindungsgemäßen Verfahren erhältlich ist. Dieses Präparat kann dann als Grundlage (natürlicher Rohstoff) für die weitere Verwendung vor allem in pharmazeutischen als auch in Lebensmittelzubereitungen dienen. So können die erfindungsgemäßen Präparate, insbesondere die Vitamin B-reichen Grassaft-Extrakte im pharmazeutischen Bereich als Tabletten, Kapseln, Pulvermischungen zum Auflösen, Brausetabletten, Fertiglösungen, orale und externe Emulsionen und Suspensionen, Injektabilia, Suppositorien, transdermale Systeme, Veterinärprodukte und Klinik-Nahrung eingesetzt werden. Im Lebensmittelbereich können die erfindungsgemäß erhältlichen Präparate in Cerealien, Milchprodukten aller Art, Fertiggerichten, flüssigen Zubereitungen aller Art, Futtermitteln für den Veterinärbereich, Müeslis und Müesliriegeln, Energieriegeln, Sportlernahrung, Seniorenkost, Instant-Formulierungen, pastösen Zubereitungen, Spaghetti, Pasta u.a. Nudelgerichten und Backmischungen vorgesehen werden, wobei sie auf Grund ihrer erhöhten Vitamin B- und gegebenenfalls Spurenelement-Gehalte besonders gut geeignet sind und zum besseren Allgemeinbef-inden der Konsumenten dieser Präparate beitragen.

Die Erfindung wird an Hand der nachfolgenden Beispiele, auf die sie selbstverständlich nicht eingeschränkt sein soll, näher erläutert.

**Beispiel 1**: Saatgutweizen wurde mit Düngelösungen folgender Zusammensetzung inkrustiert:

### Zusammensetzung mg/200 g Inkrustierungslösung:

**Tabelle 1**

| | Konzentration 1-fach | Konzentration 5-fach | Konzentration 10-fach |
|---|---|---|---|
| Natriumselenat | 1,625 mg | 8,125 mg | 16,25 mg |
| Natriummolybdat | 1,260 mg | 6,300 mg | 12,60 mg |
| Chrom-III-chlorid | 5,120 mg | 25,600 mg | 51,20 mg |
| Manganchlorid | 14,750 mg | 73,750 mg | 147,50 mg |
| Kupfergluconat | 35,700 mg | 178,500 mg | 357,00 mg |
| Ammonium-Eisen-III-citrat | 35,700 mg | 178,500 mg | 357,00 mg |
| Zinkgluconat | 78,00 mg | 394,000 mg | 788,00 mg |

Jeweils 1000g Saatgutweizen (Sorte Dekan unbehandelt (Fa. SAREA-Samenpille)) wurden mit 200 g der oben genannten Dünge-Lösung inkrustiert, d.h., die Lösung wurde auf die Oberfläche des Saatgutweizens aufgesprüht und mittels Bindemittel an der Oberfläche fixiert. Anschließend wurden die drei unterschiedlich stark besprühten Chargen des Saatgutweizens sowie eine Probe des Weizens ohne Besprühung mit der Spurenelemente-Lösung in die Erde eingebracht (429 g/m², also 199 g/0,46 m²).

Die vier Versuchs-Quadranten wurden anschließend über etwa 4 Wochen nach üblichen Anbaubedingungen mit herkömmlichem Leitungswasser besprüht, bis das Weizengras eine Höhe von etwa 25 cm erreicht hatte. Das Weizengras wurde anschließend auf einem Bodenabstand von etwa 3 cm geschnitten, nach gleichen Bedingungen zu Saft gepresst und sprühgetrocknet. Anschließend wurden das trockene Weizengrassaftpulver (WGSP) sowie die Vergleichsprobe (Weizen-Freilandanbau) auf Spurenelement-Gehalte mittels ICP-MS analysiert. Zum Vergleich wurden auch die Pressrückstände (Presskuchen) auf Spurenelement-Gehalt untersucht. Die Ergebnisse finden sich in nachfolgender Tabelle 2.

**Tabelle 2**

| | WGSP-1-Inkrustiert | WGSP 5-Inkrustiert | WGSP 10-Inkrustiert |
|---|---|---|---|
| Cr | 1,64 ± 0,08 | 1,81 ± 0,10 | 1,74 ± 0,04 |
| Mn | 106 ± 3 | 108 ± 6 | 104 ± 1 |
| Fe | 197 ± 6 | 200 ± 8 | 229 ± 1 |
| Cu | 20,5 ± 0,1 | 19,4 ± 0,6 | 21,2 ± 0,1 |
| Zn | 76 ± 1 | 70 ± 1 | 75 ± 2 |
| Se | 4,13 ± 0,05 | 19,5 ± 0,9 | 45,2 ± 0,3 |
| Mo | 12,9 ± 0,2 | 19,3 ± 0,4 | 26,5 ± 0,2 |
| | | | |

| | Presskuchen 1-Inkrustiert | Presskuchen 5-Inkrustiert | Presskuchen 10-Inkrustiert |
|---|---|---|---|
| Cr | 1, 0 ± 0, 2 | 0,6 ± 0,4 | 0,9 ± 0, 3 |
| Mn | 43 ± 2 | 43 ± 4 | 44 ± 5 |
| Fe | 93 ± 5 | 85 ± 10 | 94 ± 3 |
| Cu | 7,1 ± 0,8 | 5,8 ± 0,8 | 6,6 ± 0,3 |
| Zn | 37 ± 3 | 39,7 ± 0,9 | 45 ± 5 |
| Se | 1,1 ± 0,1 | 3,8 ± 0,4 | 8,3 ± 0,7 |
| Mo | 8,1 ± 0, 2 | 10 ± 2 | 13 ± 1 |
| | | | |

| | Weizen Freilandanbau 0045 | | |
|---|---|---|---|
| Cr | 0,58 ± 0,07 | | |
| Mn | 38 ± 1 | | |
| Fe | 57 ± 4 | | |
| Cu | 6,0 ± 0,2 | | |
| Zn | 33 ± 2 | | |
| Se | 0,36 ± 0,04 | | |
| Mo | 0,62 ± 0,01 | | |

alle Ergebnisse in mg/kg (n = 3)

Die Untersuchungen der Vitamin B-Gehalte von Weizengrassaftpulvern aus Weizensaatgut, das einmal mit Spurenelemente-Lösung angereichert wurde, aber auch ohne Vorkeimung oder Inkrustierung mit Düngelösung, zeigten keine signifikanten Einflüsse auf die Gehalte an B-Vitaminen, insbesondere Vitamin B1 und Vitamin B2 in diesem ersten Schnitt.

Im vorliegenden Beispiel wurde aber nach 1 bis 2 Wochen aus dem in der Erde verbliebenen Saatgut wiederum Weizengras wachsen gelassen und erneut geschnitten (2. Schnitt). Wird auch dieser 2. Schnitt geerntet, so wächst Weizengras auch ein drittes Mal aus dem in der Erde verbliebenen Saatgut (3. Schnitt). Eine Analyse der drei Weizengras-Schnitte ergab eine signifikante Steigerung der Vitamin B-Gehalte vom ersten bis zum dritten Schnitt, wie in der Tabelle 3 gezeigt wird.

**Tabelle 3**

| Produkte | Riboflavingehalt in mg/kg |
|---|---|
| Weizen Capo | 0,7 |
| PMN (Stamag) 4,5 Tage gekeimt | 2,2 |
| PMN Blatt (8,5 Tage) | 12 |
| Weizengrassaft von Klimakammer | 1,8 - 1,9 |
| Weizengrassaft von Feldversuch (1.Schnitt) | 1,9 - 2,2 |
| Weizengrassaft von Feldversuch (2. Schnitt) | 2,8 - 3,2 |
| Weizengrassaft von Feldversuch (3.Schnitt) | 4,1 - 4,4 |
| Weizengrassaftpulver von Klimakammer | 28,8 - 30,4 |
| Weizengrassaftpulver (1. Schnitt) | 30,4 - 35,2 |
| Weizengrassaftpulver (2. Schnitt) | 44,8 - 51,2 |
| Weizengrassaftpulver (3. Schnitt) | 65,6 - 70,4 |
| PMN-Keim | 7 , 0 - 7,5 |
| PMN-Kleie | -6,0 - 6,5 |

Diese, in der Literatur bisher noch nicht beschriebenen erhöhten Gehalte an B-Vitaminen zeigen, dass es möglich ist, durch mehrmaliges Ernten von Gräsern, insbesondere von Weizengras, die Vitamin B-Produktion im heranwachsenden Gras ohne zusätzliche Hilfsmittel anzuregen. Ein auf diese Weise gewonnenes Weizengras mit hohen Gehalten an B-Vitaminen kann durch nachfolgende technologische Verfahren (Zentrifugieren, Nanofiltrieren, Sprühtrocknen, etc.) zu natürlichen Rohstoffen mit bisher einzigartig hohen Gehalten an natürlich gewachsenen B-Vitaminen führen.

## Patentansprüche

1. Verfahren zur Herstellung Vitamin B-angereicherter Präparate aus Gräsern, **dadurch gekennzeichnet, dass** Gräser bis maximal vor der Knospungsphase wachsen gelassen werden, die Gräser geschnitten werden, die Gräser erneut wachsen gelassen werden, die Gräser erneut geschnitten werden, wobei der erneute Schnitt gesammelt wird, gegebenenfalls der Schritt des erneuten Wachsenlassens und erneuten Schneidens zumindest einmal wiederholt wird und die weiteren erneuten Schnitte gesammelt und vereinigt werden, und der erneute Schnitt oder die erneuten Schnitte zu einem Vitamin B-angereicherten Gräser-Präparat aufgearbeitet werden.

2. Verfahren nach Anspruch 1, **dadurch gekennzeichnet, dass** die Gräser aus Elektrolyt-inkrustiertem Saatgut wachsen gelassen werden.

3. Verfahren nach Anspruch 2, **dadurch gekennzeichnet, dass** das Elektrolyt-inkrustierte Saatgut durch Behandlung von Saatgut mit einer Inkrustierlösung, enthaltend Natriumselenat, Natriummolybdat, Chrom-III-chlorid, Manganchlorid, Kupfergluconat, Ammonium-Eisen-III-citrat, Zinkgluconat oder Mischungen davon, erhalten wird.

4. Verfahren nach Anspruch 3, **dadurch gekennzeichnet, dass** die Inkrustierlösung Natriumselenat, Natriummolybdat, Chrom-III-chlorid, Manganchlorid, Kupfergluconat, Ammonium-Eisen-III-citrat oder Zinkgluconat in einer Konzentration von 0,1 mg - 100 g/200 g Inkrustierlösung enthält.

5. Verfahren nach Anspruch 3 oder 4, **dadurch gekennzeichnet, dass** die Inkrustierlösung, bezogen auf 200 g Inkrustierlösung, Natriumselenat in einer Konzentration von 0,1 bis 100 mg, insbesondere 1 bis 50 mg, von Natriummolybdat 0,1 bis 100 mg, insbesondere 1 bis 50 mg, von Chrom-III-chlorid 0,5 bis 500 mg, insbesondere 1 bis 100 mg, von Manganchlorid 1 bis 1000 mg, insbesondere 10 bis 500 mg, von Kupfergluconat 1 bis 2000 mg, insbesondere 10 bis 1000 mg, von Ammonium-Eisen-III-citrat 1 bis 2000 mg, insbesondere 10 bis 1000 mg, von Kupfergluconat 1 bis 2000 mg, insbesondere 10 bis 1000 mg, und von Zinkgluconat 1 bis 2000 mg, insbesondere 10 bis 1000 mg, oder Mischungen davon enthält.

6. Verfahren nach einem der Ansprüche 1 bis 5, **dadurch gekennzeichnet, dass** Gräser von Hülsenfrüchten und Getreidegräser verwendet werden.

7. Verfahren nach einem der Ansprüche 1 bis 6, **dadurch gekennzeichnet, dass** Weizen-, Buchweizen-, Quinoa-, Mungobohnen-, Bockshornklee-, Rettich-, Alfalfa-, Mais-, Kürbis-, Roggen-, Gerste-, Reis-, Adzuki-Bohnen-, Erbsen-, Hirse-, Kichererbsen-, Kresse-, Leinsamen-, Linsen-, Senf-, Sesam-, Sojabohnen-, Sonnenblumen- und Amaranthgräser, insbesondere Weizengräser, verwendet werden.

8. Verfahren nach einem der Ansprüche 1 bis 7, **dadurch gekennzeichnet, dass** der erneute Schnitt oder die erneuten Schnitte zu einem Vitamin B-angereicherten Grassaft oder Grassaftpulver aufgearbeitet werden.

9. Vitamin B-angereichertes Gräserpräparat, erhältlich nach einem der Ansprüche 1 bis 8.

10. Pharmazeutische Zusammensetzung enthaltend ein Grassaftpräparat gemäß Anspruch 9.

11. Lebensmittelzusammensetzung enthaltend ein Vitamin B-angereichertes Gräserpräparat nach Anspruch 9.

## Claims

1. A method for producing vitamin-B-enriched preparations from grasses, **characterised in that** grasses are allowed to grow, at the longest, until before they reach their budding phase, said grasses are cut, said grasses are left to grow again, said grasses are cut again, with said further cut being collected, the step of leaving the grass to grow again and cutting it again being optionally repeated at least once, and said further cuts being collected and combined, and said further cut(s) being processed to a vitamin-B-enriched grass preparation.

2. The method according to claim 1, **characterised in that** the grasses are allowed to grow from electrolyte-encrusted seeds.

3. The method according to claim 2, **characterised in that** the electrolyte-encrusted seeds are obtained by treating seeds with an encrusting solution which contains sodium selenate, sodium molybdate, chromium-III-chloride, manganese chloride, copper gluconate, ammonium-iron-III-citrate, zink-gluconate, or mixtures thereof.

4. The method according to claim 3, **characterised in that** the encrusting solution contains sodium selenate, sodium molybdate, chromium-III-chloride, manganese chloride, copper gluconate, ammonium-iron-III-citrate or zink gluconate at a concentration of from 0.1mg to 100g/200g of encrusting solution.

5. The method according to claim 3 or 4, **characterised in that** said encrusting solution contains, relative to 200g of encrusting solution, sodium selenate at a concentration of from 0.1 to 100mg, particularly 1 to 50mg, sodium molybdate at 0.1 to 100mg, particularly 1 to 50mg, chromium-III-chloride at 0.5 to 500mg, particularly 1 to 100mg, manganese chloride at 1 to 1000mg, particularly 10 to 500mg, copper gluconate at 1 to 2000mg, particularly 10 to 1000mg, ammonium-iron-III-citrate at 1 to 2000mg, particularly 10 to 1000mg, copper gluconate at 1 to 2000mg, particularly 10 to 1000mg, and zink gluconate at 1 to 2000mg, particularly 10 to 1000mg, or mixtures thereof.

6. The method according to any one of claims 1 to 5, **characterised in that** the grasses of leguminous plants and grains are used.

7. The method according to any one of claims 1 to 6, **characterised in that** the grasses of wheat, buckwheat, quinoa, mungo beans, fenugreek, radish, alfalfa, corn (maize), pumpkin, rye, barley, rice, adzuki beans, peas, millet, chick-peas, cress, linseed, lentils, mustard, sesame, soy beans, sunflowers, and amaranth, particularly wheat grasses, are used.

8. The method according to any one of claims 1 to 7, **characterised in that** said further cut(s) is (are) processed to a vitamin-B-enriched grass juice or grass-juice powder.

9. A vitamin-B-enriched grass preparation, obtainable according to any one of claims 1 to 8.

10. A pharmaceutical composition containing a grass-juice preparation according to claim 9.

11. A food composition containing a vitamin-B-enriched grass preparation according to claim 9.

## Revendications

1. Procédé pour la production de préparations enrichies en vitamine B à partir d'herbes, **caractérisé en ce qu'**on fait pousser les herbes jusqu'au maximum avant la phase de bourgeonnement, on coupe les herbes, on fait à nouveau pousser les herbes, on coupe à nouveau les herbes en recueillant la nouvelle coupe, éventuellement on répète au moins une fois la nouvelle pousse et la nouvelle coupe et on recueille et réunit les autres nouvelles coupes, et on traite la nouvelle coupe ou les nouvelles coupes pour obtenir une préparation d'herbes enrichie en vitamine B.

2. Procédé selon la revendication 1, **caractérisée en ce qu'**on fait pousser les herbes à partir de semences à incrustation d'électrolyte.

3. Procédé selon la revendication 2, **caractérisé en ce que** la semence à incrustation d'électrolyte est obtenue par traitement de la semence par une solution d'incrustation contenant du sélénate de sodium, du molybdate de sodium, du chlorure de chrome-III, du chlorure de manganèse, du gluconate de cuivre, du citrate d'ammonium et de fer-III, du gluconate de zinc ou des mélanges de ceux-ci.

4. Procédé selon la revendication 3, **caractérisé en ce que** la solution d'incrustation contient du sélénate de sodium, du molybdate de sodium, du chlorure de chrome-III, du chlorure de manganèse, du gluconate de cuivre, du citrate d'ammonium et de fer-III ou du gluconate de zinc à une concentration de 0,1 mg à 100 g/200 g de solution d'incrustation.

5. Procédé selon la revendication 3 ou 4, **caractérisé en ce que** la solution d'incrustation contient, par rapport à 200 g de solution d'incrustation, du sélénate de sodium à une concentration de 0,1 à 100 mg, en particulier de 1 à 50 mg, du molybdate de sodium à une concentration de 0,1 à 100 mg, en particulier de 1 à 50 mg, du chlorure de chrome-III à une concentration de 0,5 à 500 mg, en particulier de 1 à 100 mg, du chlorure de manganèse à une concentration de 1 à 1 000 mg, en particulier de 10 à 500 mg, du gluconate de cuivre à une concentration de 1 à 2 000 mg, en particulier de 10 à 1 000 mg, du citrate d'ammonium et de fer-III à une concentration de 1 à 2 000 mg, en particulier de 10 à 1 000 mg, du gluconate de cuivre à une concentration de 1 à 2 000 mg, en particulier de 10 à 1 000 mg et du gluconate de zinc à une concentration de 1 à 2 000 mg, en particulier de 10 à 1 000 mg, ou des mélanges de ceux-ci.

6. Procédé selon l'une quelconque des revendications 1 à 5, **caractérisé en ce qu'**on utilise des herbes de légumineuses ou de céréales.

7. Procédé selon l'une quelconque des revendications 1 à 6, **caractérisé en ce qu'**on utilise des herbes de blé, sarrasin, quinoa, haricot mungo, fenugrec, radis noir, luzerne, maïs, courge, seigle, orge, riz, haricot adzuki, pois, millet, pois chiche, cresson, lin, lentille, moutarde, sésame, soja, tournesol et amarante, en particulier des herbes de blé.

8. Procédé selon l'une quelconque des revendications 1 à 7, **caractérisé en ce qu'**on traite la nouvelle coupe ou les nouvelles coupes pour obtenir un jus d'herbes ou une poudre de jus d'herbes enrichis en vitamine B.

9. Préparation à base d'herbes enrichie en vitamine B, pouvant être obtenue selon l'une quelconque des revendications 1 à 8.

10. Composition pharmaceutique contenant une préparation de jus d'herbes selon la revendication 9.

11. Composition de produit alimentaire contenant une préparation à base d'herbes enrichie en vitamine B selon la revendication 9.
